# EUROPÄISCHE PATENTSCHRIFT

(11) **EP 3 558 177 B1**
(45) Veröffentlichungstag und Bekanntmachung des Hinweises auf die Patenterteilung: **12.08.2020**
(21) Anmeldenummer: 18702206.6
(22) Anmeldetag: 25.01.2018
(51) Int. Cl.: A61F 5/455

(54) **HYGIENEPRODUKT, INSBESONDERE MENSTRUATIONSTASSE MIT ERGONOMISCHER FORM**
HYGIENE PRODUCT, IN PARTICULAR MENSTRUAL CUP WITH AN ERGONOMIC SHAPE
PRODUIT HYGIÉNIQUE, NOTAMMENT COUPE MENSTRUELLE AYANT UNE FORME ERGONOMIQUE

(30) Priorität: 25.01.2017 DE 102017101383
(43) Veröffentlichungstag der Anmeldung: 30.10.2019
(73) Patentinhaber: Fun Factory GmbH, 28197 Bremen (DE)
(72) Erfinder: BAUER, Dirk, 28197 Bremen (DE)
(74) Vertreter: Eisenführ Speiser
(86) Internationale Anmeldenummer: PCT/EP2018/051852
(87) Internationale Veröffentlichungsnummer: WO 2018/138207

(56) Entgegenhaltungen:
- WO-A1-2006/058409
- US-A- 2 534 900
- US-A1- 2013 110 060
- FUN FACTORY GmbH: "FUN CUP by FUN FACTORY Produktvideo - deutsch", , 29. August 2017 (2017-08-29), XP054978191, Gefunden im Internet: URL:https://www.youtube.com/watch?v=YT7Np8 tTJS4 [gefunden am 2018-03-16]

## Beschreibung

Die Erfindung betrifft ein Hygieneprodukt, insbesondere Menstruationstasse, mit einem Tassenkörper, der eine Tassenwand aufweist, die mit einer inneren Oberfläche einen Aufnahmeraum definiert, wobei der Tassenkörper an einem ersten Ende eine in den Aufnahmeraum mündende Öffnung mit einem Rand und an dem zweiten Ende einen der Öffnung gegenüberliegenden Boden aufweist, und wobei der Tassenkörper eine im Allgemeinen kegelige Form ausweist, sich zum Boden hin verjüngt und in einer Spitze endet.

Menstruationstassen der eingangs genannten Art existieren auf dem Markt in vielfältiger Weise. Im Allgemeinen zeichnen sich solche Menstruationstassen durch einen Tassenkörper aus, der im Allgemeinen kegelig oder glockenförmig gebildet ist, an einer ersten Seite eine Öffnung aufweist und an einer zweiten Seite in eine Spitze mündet. Die Spitze ist üblicherweise mit einem stiftförmigen Vorsprung oder Stil versehen, der als Handgriff dient. Der die Öffnung begrenzende Rand ist üblicherweise abgerundet und verstärkt, um den Tassenkörper aufzuspreizen.

Der Stift oder auch Stiel ist in der Regel mit einer Strukturierung, beispielsweise in Form von umlaufenden Rippen versehen, um diesen besser greifen zu können. Auch bekannt sind Stifte, an denen Fäden angeordnet sind, Ringe oder andere Griffelemente.

Übliche Menstruationstassen sind dabei rotationssymmetrisch ausgebildet. Dies hat den Zweck und Vorteil, dass das Einsetzen vereinfacht ist. Beim Einsetzen wird die Menstruationstasse zusammengedrückt, das heißt gefaltet, sodass sie an dem Rand eine im Wesentlichen U-förmige Kontur aufweist. Anschließend wird sie in die weibliche Scheide in Richtung der Gebärmutter eingeführt. Aufgrund des verstärkten Rands gewinnt die Menstruationstasse dann ihre ursprüngliche Form wieder und ist in der Lage, Flüssigkeit in dem Aufnahmeraum aufzunehmen. Zum Wechseln oder Reinigen der Menstruationstasse wird diese dann mit zwei Fingern an dem Stift gegriffen und herausgezogen. Zur Vermeidung eines Unterdrucks beim Rückgewinnen der Form oder beim Entfernen der Menstruationstasse weist diese in der Regel Lüftungsöffnungen auf, die im Bereich des oberen Rands angeordnet sind.

Eine derartige Menstruationstasse ist in WO 2006/058409 A1 offenbart.

Eine ähnliche Menstruationstasse mit einem etwas längeren Stift, der sich auch zum Abschneiden und somit Anpassen der Stiftlänge an die jeweilige Person eignet, ist in US D746,452 S offenbart.

Eine Menstruationstasse die mit einem ringförmigen Griff versehen ist, der als Handgriff dient, ist in der US 2013/110060 A1 offenbart.

Eine sehr frühe Veröffentlichung einer Menstruationstasse ist in US 2,534,900 offenbart.

Die dort vorgeschlagene Menstruationstasse weist keine vollständig rotationssymmetrische Form auf, sondern hat an einer Seite in der Richtung der unteren Spitze einen Vorsprung. Ein Rücken des im Wesentlichen kegeligen oder glockenförmigen Tassenkörpers aus US 2,534,900 erstreckt sich im Wesentlichen gerade in Richtung der Spitze und erst dann in einer recht scharfen Krümmung in Richtung der Spitze. So wird der Vorsprung nach Art einer Art Finne gebildet. Diese soll ein Verdrehen der Menstruationstasse verhindern.

Eine weitere Menstruationstasse, die den Sitz verbessern soll, ist in CN 204971787 U offenbart. Die dort offenbarte im Wesentlichen rotationssymmetrische Menstruationstasse ist im Bereich der oberen Öffnung abgeschrägt. Der Rand ist dabei nicht verstärkt ausgebildet wie bei den weiteren im Stand der Technik bekannten Menstruationstassen, sondern weist einen sich radial nach innen erstreckenden weichen Kragen auf. Durch diesen Kragen soll ein Herausfließen von aufgenommener Flüssigkeit vermieden werden.

Neben dem Problem der richtigen Positionierung besteht ein weiteres Problem bei der Entnahme der Menstruationstasse. Wird diese beispielsweise nur an einem dünnen Stift herausgezogen, besteht die Gefahr, dass diese einer Bedienerin nach dem Entnehmen entgleitet oder am Stift umknickt und somit aufgenommene Flüssigkeit leicht verschüttet werden kann.

Auch diesem Problem wird teilweise durch den in CN 204971787 U offenbarten radial nach innen weisenden Kragen begegnet.

Andere Lösungen schlagen ein Ventil oder eine Entnahmeöffnung im Bereich der Spitze vor. Dies ist beispielsweise in CN 106073976 A oder WO 2014/056257 A1 vorgeschlagen. Bei solchen Menstruationstassen ist theoretisch ein Entleeren auch im eingesetzten Zustand möglich, nämlich durch Öffnen des Ventils.

Aber auch solche Lösungen haben sich als unpraktisch erwiesen, insbesondere da sich das Ventil versehentlich öffnen kann.

Aufgabe der vorliegenden Erfindung ist es also, ein Hygieneprodukt, insbesondere Menstruationstasse, anzugeben, die im Sitz verbessert ist, in der Handhabung einfach und sich problemlos und sicher entnehmen lässt.

Diese Aufgabe wird bei einem Hygieneprodukt der eingangs genannten Art dadurch gelöst, dass der Tassenkörper rotationsunsymmetrisch ist und eine Zentralachse des Tassenkörpers gekrümmt verläuft.

Die Erfindung basiert auf der Idee, dass für einen guten Sitz des Hygieneprodukts innerhalb der Scheide eine rotationsunsymmetrische Form besser geeignet ist. Hierdurch lässt sich die Außenkontur des Hygieneprodukts besser an die anatomischen Gegebenheiten anpassen und der Sitz optimieren. Dabei hat sich gezeigt, dass es nicht ausreicht, nur einen äußerlichen Steg vorzusehen, wie dies im Stand der Technik bekannt ist, oder die Öffnung schräg bezogen auf eine Zentralachse anzuordnen, wie dies ebenfalls im Stand der Technik bekannt ist. Die Erfindung basiert vielmehr auf der Idee, dass die Menstruationstasse in sich insgesamt gekrümmt ausgebildet sein muss, das heißt, dass die Zentralachse, die sich zentral durch den Körper des Hygieneprodukts erstreckt, gekrümmt ist. Die Zentralachse verläuft zunächst von dem Zentrum der Öffnung, durch den Aufnahmeraum und schließlich durch die Spitze. Als Zentralachse ist hier die jeweilige Verbindung zwischen Zentren von Schnitten entlang der Zentralachse von dem Zentrum der Öffnung bis zur Spitze zu verstehen.

Die Gefahr eines Verdrehens des Hygieneprodukts in der Benutzung besteht bei der Ausführung der Erfindung tatsächlich nicht. Die rotationsunsymmetrische Form als solche verhindert bereits ein Verdrehen, da aufgrund der Krümmung die äußere Kontur des Hygieneprodukts an die Innenkontur der Scheide angepasst ist. Dadurch findet vielmehr eine Selbstpositionierung in der Bewegung statt.

Auch besteht das Problem des Positionierens nicht. Zwar ist es bei rotationssymmetrischen Menstruationstassen irrelevant, an welcher umfänglichen Stelle diese zum Einsetzen eingeknickt wird, allerdings ist bei einer rotationsunsymmetrischen Menstruationstasse für die Bedienerin klar, an welcher Stelle diese eingedrückt werden soll.

Auch das Entnehmen ist vereinfacht, da die Zentralachse gekrümmt ist. Durch die Krümmung ist es möglich, die Ebene der Öffnung im Wesentlichen horizontal zu halten, während die Menstruationstasse entnommen wird. Beim Entnehmen verläuft eine Zentralachse der Scheide ebenfalls gekrümmt, sodass die Zentralachse der Scheide und die Zentralachse des Tassenkörpers im Wesentlichen koaxial verlaufen. Die Entnahme ist auf diese Weise wesentlich vereinfacht, und ein Verschütten kann vermieden werden. Die Zentralachse ist vorzugsweise über einen Abschnitt in einem Bereich von 30° bis 90°, vorzugsweise 45° bis 80° gekrümmt. Ein Krümmungsradius liegt vorzugsweise in einem Bereich von 2cm bis 30cm, wobei der Krümmungsradius entlang der Zentralachse variieren kann.

Der Tassenkörper endet dabei an der Spitze und weist vorzugsweise keinen Stift oder Stiel auf. Es hat sich gezeigt, dass durch einen Stiel das Hygieneprodukt zwar leichter zu ergreifen ist, aber die Gefahr des Verkippens beim Herausnehmen besteht. Dies umso mehr, wenn der Stiel nur am äußersten Ende ergriffen wird. Weist das Hygieneprodukt jedoch keinen Stiel auf, ist eine Bedienerin dazu veranlasst, das Hygieneprodukt an der Spitze des Tassenkörpers zu ergreifen, wodurch ein sichererer und kippunempfindlicherer Griff erreicht wird.

Gemäß einer ersten bevorzugten Ausführungsform weist der Tassenkörper eine Symmetrieebene auf und ist bezogen auf diese Symmetrieebene spiegelsymmetrisch ausgebildet. Der Tassenkörper ist rotationsunsymmetrisch, aber spiegelsymmetrisch. Dies entspricht auch der natürlichen Form der inneren Scheidenwand, sodass eine Anpassung des Tassenkörpers an die Scheide verbessert ist und somit der Sitz des Hygieneprodukts in der Benutzung verbessert ist. Ferner wird hierdurch auch das Positionieren vereinfacht, da ein Falten der Menstruationstasse vereinfacht ist. Die Faltung wird vorzugsweise entlang der Symmetrieachse vorgenommen, sodass in der U-förmigen Faltungsposition die Symmetrieebene zwischen den beiden Schenkeln des U verläuft.

Weiterhin ist bevorzugt, dass ein erster Abschnitt der Tassenwand entlang der Symmetrieebene von dem Rand der Öffnung bis zur Spitze an der Außenseite im Wesentlichen teilkreisförmig verläuft. Dieser erste Abschnitt der Tassenwand kann auch als Rücken bezeichnet werden, da er im eingesetzten Zustand in Richtung des Rückens der Benutzerin zeigt. Hierdurch wird die Krümmung unterstützt und ein ergonomischer Sitz des Hygieneprodukts erreicht.

Weiterhin ist bevorzugt, dass ein zweiter Abschnitt der Tassenwand entlang der Symmetrieebene von dem Rand der Öffnung bis zur Spitze im Wesentlichen eine konvex-konkave Form definiert. Das heißt, der zweite Abschnitt der Tassenwand verläuft zunächst in einer konvexen Form und anschließend konkav bis zur Spitze. Auch hierdurch wird die Krümmung unterstützt. Der Tassenkörper erhält so eine leicht bauchige oder glockenförmige Form, ist aber insgesamt kegelig ausgebildet. Die Form des Tassenkörpers kann auch verglichen werden mit einem Tropfen, der zum Tropfenende hin verkrümmt verläuft.

Weiterhin ist bevorzugt, dass der Tassenkörper eine bauchige Form hat, indem sich der Tassenkörper im Bereich der Öffnung leicht verjüngt. Hierdurch wird einerseits die Positionierung vereinfacht, da die Position nicht nur durch den Rand, sondern auch und im Wesentlichen durch die Seitenwände gehalten wird. Andererseits wird auch ein Verschütten von aufgenommener Flüssigkeit effektiver vermieden, indem sich der Tassenkörper im Bereich der Öffnung leicht verjüngt.

Gemäß einer weiteren bevorzugten Ausführungsform weist die Spitze eine im Allgemeinen kegelige Form auf. Hierdurch ist die Spitze an sich zunächst schwerer greifbar als ein gerader Stiel. Allerdings führt eine kegelige Spitze dazu, dass eine Bedienerin dazu angehalten ist, den Tassenkörper fest zu greifen, wodurch wiederum ein Verkippen nach dem Entnehmen vermieden wird. Durch eine zunächst erschwerte Greifbarkeit des Tassenkörpers wird somit im Endeffekt ein sichereres Greifen erreicht und ein Verkippen verhindert.

Bevorzugt ist die Spitze als Verdickung in der Tassenwand ausgebildet. Hierdurch ist es möglich, die Spitze fest zu greifen, ohne diese zu sehr zusammenzudrücken. Ferner ist es hierdurch möglich, die Tassenwand oberhalb der Spitze, das heißt benachbart zu der Spitze zwischen Spitze und der Öffnung zusammenzudrücken und so die Verdickung, die die Spitze bildet, zu hintergreifen. Hierdurch wird ein besonders sicheres Entnehmen und eine besonders gute Verkippsicherung erreicht.

Die Verdickung entspricht vorzugsweise in etwa dem Drei- bis Sechsfachen, vorzugsweise dem Vier- bis Fünffachen der Wandstärke der Tassenwand. Gemessen wird die Stärke der Verdickung an der dicksten Stelle der Verdickung. Es hat sich gezeigt, dass eine Verdickung in diesem Bereich zu einem besonders sicheren und einfachen Greifen führt, ohne jedoch in der Benutzung störend zu wirken.

Besonders bevorzugt weist die Spitze eine Länge in einem Bereich von 5 bis 12 mm auf. Ein solcher Bereich hat sich als geeignet gezeigt, um die Spitze, die durch die Verdickung gebildet ist, gut hintergreifbar zu gestalten. Eine Bedienerin kann die verdickte Spitze mit einer solchen Größe gut ertasten und diese hintergreifen, um so das Hygieneprodukt zu entnehmen.

Vorzugsweise geht die Tassenwand knickfrei in die Spitze über. Auch hierdurch wird die Stabilität erhöht. Im Stand der Technik sind eine Vielzahl von Menstruationstassen bekannt, die zwar eine kegelige oder glockenförmige Form aufweisen, bei denen sich der Stiel aber mit einem Knick, insbesondere einem Knick in etwa im rechten Winkel, aus der Tassenwand im Bereich der Spitze erstreckt. Durch einen knickfreien Übergang wird einerseits die Steifigkeit in dem Bereich leicht erhöht, andererseits wird auch die Reinigung des Hygieneprodukts vereinfacht.

In einer bevorzugten Weiterbildung weist die Tassenwand äußerlich im Bereich der Spitze eine Strukturierung auf. Da die Menstruationstasse im Bereich der Spitze ergriffen wird, um diese zu entnehmen, ist eine solche Strukturierung bevorzugt, um die Reibung zwischen den ergreifenden Fingern und dem Tassenkörper zu erhöhen. Bevorzugt ist die restliche Oberfläche der Tassenwand äußerlich ohne Strukturierung ausgebildet. Im Stand der Technik sind auch Tassen bekannt, die über den gesamten Körper verteilt äußerlich Rippen aufweisen. Allerdings führen solchen Strukturierungen, die nicht zum Ergreifen genutzt werden, tendenziell zu einem erschwerten Herausnehmen. Daher ist bevorzugt, dass die Tassenwand äußerlich ausschließlich im Bereich der Spitze eine Strukturierung aufweist.

Die Strukturierung ist vorzugsweise als eine Vielzahl an Vorsprüngen ausgebildet. Zwar sind auch umlaufende Rippen denkbar, allerdings können diese zu einer Abdichtung des Tassenkörpers gegen die innere Oberfläche der Scheide führen, was wiederum eine Unterdruckerzeugung beim Herausnehmen nach sich ziehen kann. Die einzelnen Vorsprünge sind vorzugsweise separiert. In einer Variante sind die Vorsprünge jeweils ringförmig ausgebildet. Das heißt, im Bereich der Spitze ist eine Vielzahl von Ringen, beispielsweise Kreisen, oder anderen Formen erhaben ausgebildet. In einer besonders bevorzugten Variante sind die Vorsprünge ringförmig ausgebildet, und haben eine Form, die Kernen einer Erdbeere ähnelt. Der Tassenkörper als solcher weist eine in etwa erdbeerförmige Form auf, und durch Ausbildung der Vorsprünge als Kerne der Erdbeere wird diese Assoziation noch verstärkt. Hierdurch erhält das Hygieneprodukt zusätzlich ein ansprechendes Aussehen, das spielerisch wirkt und weniger Assoziationen an Sanitärprodukte weckt.

Gemäß einer weiteren bevorzugten Ausführungsform ist der Rand der Öffnung verstärkt. Eine solche Verstärkung dient insbesondere dazu, den Tassenkörper nach dem Positionieren in eine aufgespreizte Form zu bringen, sodass die Öffnung tatsächlich offensteht, sodass der Aufnahmeraum Flüssigkeit aufnehmen kann. Ferner dient die Verstärkung dazu, den Tassenkörper in dieser Position zu halten, sodass auch eine Positionierung der Menstruationstasse in der Scheide erhalten bleibt.

Vorzugsweise ist diese Verstärkung als Verdickung in der Tassenwand ausgebildet. Die Verdickung erstreckt sich vorzugsweise von der Tassenwand radial nach innen, sodass der Aufnahmeraum insgesamt bauchig ausgebildet ist. Ein radial nach außen vorstehender Ring hat sich hingegen eher als nachteilig erwiesen.

Vorzugsweise ist der Rand so ausgebildet, dass er zum Aufnahmeraum hin schräg abfällt. Dies führt zu einer verbesserten Flüssigkeitsaufnahme. Der Rand kann in dem Abschnitt, in dem er in die äußere Wand der Tasse übergeht, einen relativ kleinen Radius aufweisen, um so ein dichtes Anliegen an der Haut zu ermöglichen. Hierdurch wird eine Dichtigkeit erreicht, ohne gleichzeitig einen zu hohen Druck aufzubringen. Auch hierdurch wird der Tragekomfort wesentlich verbessert.

Vorzugsweise ist die Öffnung im Wesentlichen kreisförmig. Eine kreisförmige Öffnung bietet im Fall der Verstärkung des Rands eine relativ hohe Stabilität verglichen mit anderen Formen und entspricht auch den anatomischen Gegebenheiten am Positionierungsort.

In einer weiteren bevorzugten Ausführungsform weist die Tassenwand zwischen dem Rand der Öffnung und der Spitze eine im Wesentlichen konstante Wandstärke auf. Durch eine im Wesentlichen konstante Wandstärke wird auch eine im Wesentlichen gleichmäßige Steifigkeit erreicht, und ein angenehmes Anliegen und der Tragekomfort sind verbessert. Die einzigen versteiften Bereiche sind in dieser Ausführungsform der Rand und die Spitze.

Vorzugsweise ist der Tassenkörper einstückig ausgebildet. Durch eine einstückige Ausbildung ist der Tragekomfort verbessert und auch die Reinigung des Produkts ist vereinfacht. Es sind keine Nahtstellen, Fugen oder dergleichen zwischen verschiedenen Bauteilen vorgesehen, wodurch die Reinigung wesentlich vereinfacht ist.

In einer besonders bevorzugten Ausführungsform ist der Tassenkörper aus einem Silikon mit einer Shore-Härte in einem Bereich von 18 bis 60, insbesondere 30 bis 50, vorzugsweise 35 bis 45 gebildet. Silikon eignet sich besonders zur Bildung einer Menstruationstasse, da einerseits Silikon besonders gut hautverträglich ist, andererseits in seiner Struktur eine ausreichende Flexibilität aber auch Steifigkeit bietet, um ein Positionieren zu ermöglichen. Die Dauerhaftigkeit des Silikons ist hoch, sodass das Hygieneprodukt häufig wiederverwendet werden kann. Eine Shore-Härte insbesondere in einem Bereich von 35 bis 45 hat sich als besonders geeignet erwiesen, um den Zielkonflikt zwischen der Steifigkeit und der Elastizität bzw. Flexibilität des Hygieneprodukts möglichst aufzulösen. Besonders bevorzugt ist das Silikon medizinisches Silikon. Damit ist es noch besser hautverträglich.

Vorzugsweise ist der Tassenkörper aus einem Spritzgusssilikon oder Flüssigsilikon gebildet. Insbesondere kommen Materialien wie LSR (Liquid Silicon Rubber), HTV (hochtemperaturvernetzter Silikonkautschuk), oder RTV (raumtemperaturvernetzter Silikon-Kautschuk) zum Einsatz. Auch eignen sich TPE oder Gummi.

Die Tassenwand hat vorzugsweise eine Wandstärke in einem Bereich von 0,8 mm bis 8,0 mm, vorzugsweise 1,0 mm bis 3,0 mm, weiter vorzugsweise 1,5 mm bis 2,5 mm. Besonders geeignet haben sich Wandstärken im Bereich von etwa 2,0 mm gezeigt. Hierdurch lassen sich bei entsprechender Wahl des Materials, insbesondere Silikonmaterials, Rückstellkräfte erzielen, die dazu führen, dass das Hygieneprodukt einerseits angenehm getragen werden kann, andererseits sowohl seine Position als auch einen nichtkollabierten Zustand aufrecht zu erhalten, der wichtig ist, um die Funktion ausreichend auszuführen.

Vorzugsweise weist die Tassenwand eine Rückstellkraft gegen ein Zusammendrücken in einem Bereich von 4 N bis 15 N, vorzugsweise 5 N bis 10 N, weiter bevorzugt 6 N bis 10 N auf. Der Rand der Tassenwand kann darüber hinaus eine Rückstellkraft gegen ein Zusammendrücken in einem Bereich von 6 N bis 15 N aufweisen. Mit Rückstellkraft gegen ein Zusammendrücken ist die Kraft gemeint, die erforderlich ist um den Tassenkörper an zwei sich gegenüberliegenden Seiten zusammenzudrücken, sodass sich die gegenüberliegenden Seiten gerade berühren.

Nachstehend wird die Erfindung anhand eines Ausführungsbeispiels unter Bezugnahme auf die beiliegenden Figuren näher erläutert. Dabei zeigen:
- Fig. 1: eine erste perspektivische Ansicht des Hygieneprodukts,
- Fig. 2: eine zweite perspektivische Ansicht des Hygieneprodukts aus Fig. 1,
- Fig. 3: eine Seitenansicht des Hygieneprodukts aus Fig. 1,
- Fig. 4: eine Schnittdarstellung des Hygieneprodukts aus Fig. 3 entlang der Symmetrieebene,
- Fig. 5: eine Sicht auf den Rücken, von links in Fig. 3 auf das Hygieneprodukt,
- Fig. 6: eine Draufsicht auf das Hygieneprodukt aus Fig. 3 von oben, und
- Fig. 7: eine Ansicht wie in Figur 4, zur Illustration der Krümmung der Zentralachse.

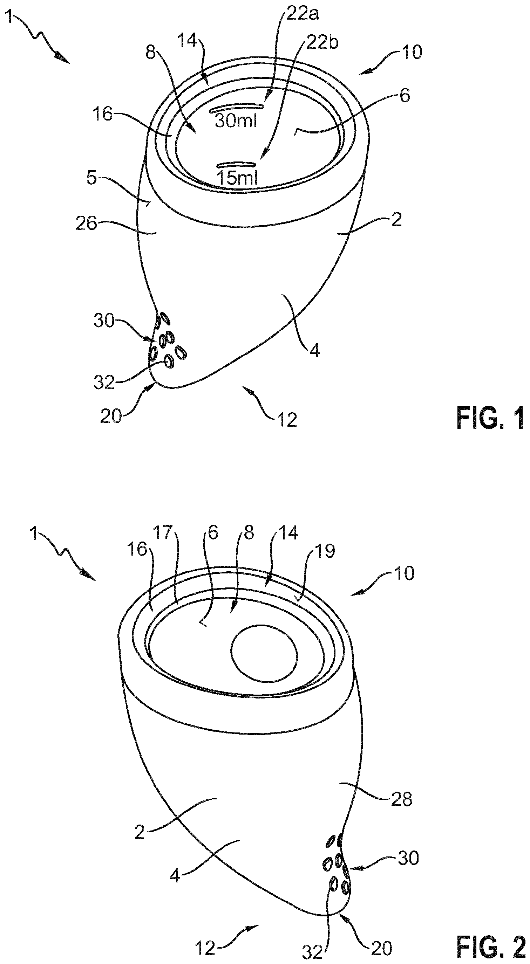

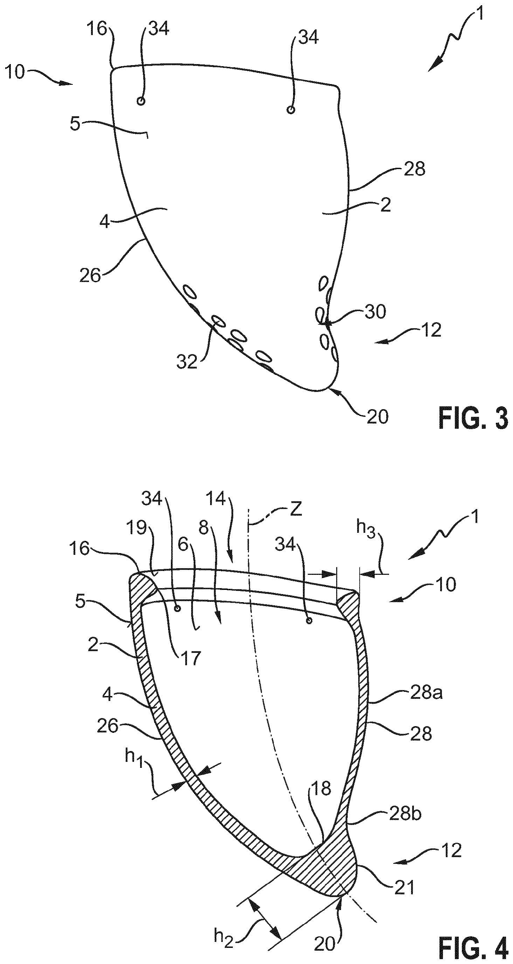

Gemäß Fig. 1 weist ein Hygieneprodukt, das hier als Menstruationstasse 1 ausgebildet ist, einen Tassenkörper 2 auf, der eine Tassenwand 4 hat, die mit einer inneren Oberfläche 6 einen Aufnahmeraum 8 definiert. Der Tassenkörper 2 weist ein erstes Ende 10 und ein zweites Ende 12 auf. An dem ersten Ende 10 hat der Tassenkörper 2 eine Öffnung 14, die von einem Rand 16 begrenzt wird. Am gegenüberliegenden Ende 12 weist der Tassenkörper 2 einen Boden 18 auf (vgl. Fig. 4).

Insgesamt ist der Tassenkörper 2 in einer kegeligen Form oder glockenförmigen Form gebildet und verjüngt sich von der Öffnung 14 in Richtung des zweiten Endes 12 und mündet dort in eine Spitze 20.

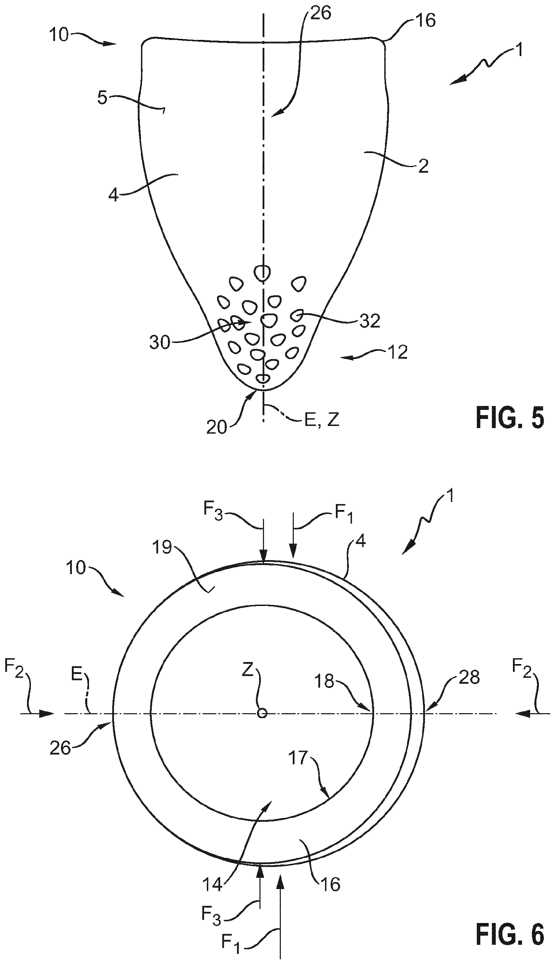

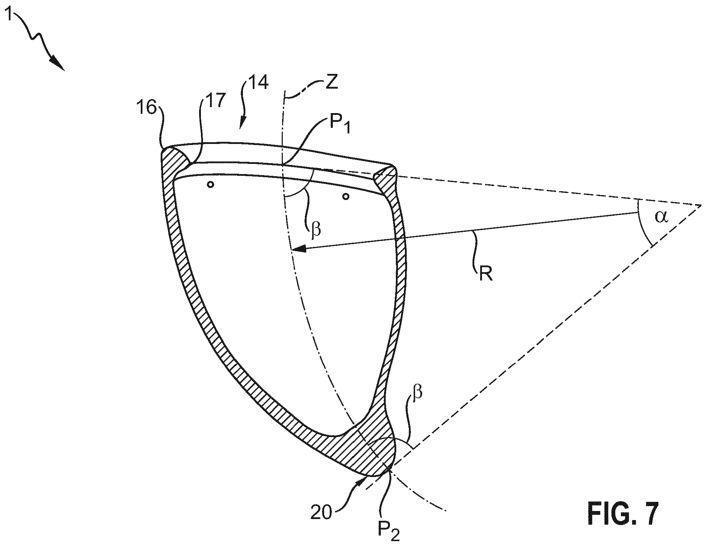

Wie sich insbesondere aus Fig. 1 bis 4 ergibt, ist dabei der Tassenkörper 2 rotationsunsymmetrisch, aber spiegelsymmetrisch. Die Zentralachse Z (vgl. Fig. 4 und 7) verläuft gekrümmt, liegt aber in der Symmetrieebene E (vgl. Fig. 6). Die Zentralachse Z verläuft zunächst durch das Zentrum der Öffnung 14, dann durch den Aufnahmeraum 8 und schließlich aus dem proximalen Ende der Spitze 20 hinaus. Bei rotationssymmetrischen Tassenkörpern ist die Zentralachse hingegen gerade.

Durch diese Krümmung des Tassenkörpers ist der Tassenkörper 2 wesentlich besser an die Anatomie der weiblichen Scheide angepasst als bisherige Menstruationstassen 1.

Der Verlauf der Krümmung er Zentralachse Z ist anhand von geometrischen Beziehungen in Figur 7 dargestellt. Die Zentralachse Z verläuft von einem ersten Schnittpunkt P₁ mit einer Ebene der Öffnung 14, die sich durch den Kamm 17 des Rands 16 erstreckt, bis zu einem zweiten Schnittpunkt P₂, dem proximalen Punkt der Spitze 20. Die Ebenen, die an diesen Punkten P₁ und P₂ senkrecht auf die Zentralachse Z stehen (vgl. Winkel β), schließen einen Winkel α miteinander ein. Der Winkel α liegt in einem Bereich von 20° bis 90°, und im vorliegenden Ausführungsbeispiel etwa im Bereich von 45°. Der Krümmungsradius R kann entlang des Abschnitts der Zentralachse Z zwischen den Punkten P₁ und P₂ variieren, und liegt im Mittel in diesem Ausführungsbeispiel bei etwa 8,5cm. Der Radius R ist aber abhängig von der Größe der Menstruationstasse 1, und kann im Mittel in einem Bereich von 5 cm bis 20cm, oder auch außerhalb dieses Bereichs liegen.

Der Aufnahmeraum 8 dient zur Aufnahme von Flüssigkeit, wenn die Menstruationstasse 1 zur Benutzung eingeführt ist. Auf der inneren Oberfläche 6 sind in diesem Ausführungsbeispiel Maßangaben 22a, 22b vorgesehen (vgl. Fig. 1), die zum Bestimmen der in dem Aufnahmeraum 8 aufgenommen Flüssigkeitsmenge dienen.

Der Aufnahmeraum 8 ist insgesamt bauchig ausgebildet und wird im Bereich der Öffnung 14 durch den Rand 16 begrenzt. Der Rand 16 ist verstärkt ausgebildet, als Verdickung, die sich radial nach innen erstreckt. Insofern hat der Rand 16 einen radial vorspringenden Kamm 17, der eine radiale Einschnürung darstellt. Eine Fläche 19, die vom Rand 16 bis zum Kamm 17 verläuft, also im Wesentlichen von der äußeren Mantelfläche 5 der Tassenwand 4 bis zum Kamm 17, ist schräg abfallend mit Bezug auf die Zentralachse Z ausgebildet. Hierdurch wird sichergestellt, dass mit Bezug auf die Fig. 3 und 4 von oben herablaufende Flüssigkeit sicher im Aufnahmeraum 8 aufgenommen wird und nicht außerhalb der Menstruationstasse 1 im Bereich der Mantelfläche 5 vorbeiläuft.

Durch die Verdickung in dem Rand 16 wird auch eine Stabilisierung der Form des Tassenkörpers 2 erreicht. Wie sich insbesondere aus Fig. 6 ergibt, weist die Öffnung 14 eine im Wesentlichen kreisförmige Kontur auf. Die Verdickung in dem Bereich des Rands dient dazu, diese Öffnung 14 aufzuspreizen, sodass die Menstruationstasse 1 funktionsfähig ist. Es ist wichtig, dass die Menstruationstasse 1 im eingesetzten Zustand nicht kollabiert, sondern möglichst aufgespreizt ist, sodass Flüssigkeit effektiv in dem Aufnahmeraum 8 aufgefangen werden kann.

Der Boden 18 ist geschlossen und weist keine Durchtrittsöffnung auf. In Abkehr zum Stand der Technik weist die Menstruationstasse 1 gemäß dem vorliegenden Ausführungsbeispiel kein Ventil oder dergleichen auf.

Von dem Rand 16 aus erstreckt sich die Tassenwand 4 in Richtung der Spitze 20 und mündet in dieser. Dabei geht die Tassenwand 4 knickfrei in die Spitze 20 über, sowohl an der inneren Oberfläche 6 als auch an der äußeren Mantelfläche 5. Ein erster Abschnitt 26 der Tassenwand 4 entlang der Symmetrieebene E von dem Rand 16 der Öffnung 14 bis zur Spitze 20 verläuft im Wesentlichen teilkreisförmig. Wie sich insbesondere aus den Fig. 3 und 4 entnehmen lässt, geht die Tassenwand 4 in dem Abschnitt 26 stetig in die Spitze 20 über; sie weist keine Unstetigkeit auf. Auch die Steigung bleibt im Wesentlichen konstant, jedenfalls findet keine Änderung der Steigungsrichtung statt. Der Abschnitt 26 ist vollständig konvex gebildet.

Auf der gegenüberliegenden Seite des Tassenkörpers 2 entlang der Symmetrieebene E weist die Tassenwand 4 einen Abschnitt 28 auf. Der Abschnitt 28 ist korrespondierend zum Abschnitt 26 ausgebildet und erstreckt sich ebenfalls von dem Rand 16 bis zur Spitze 20. Der Abschnitt 28 weist einen ersten Teilabschnitt 28a, der konvex gebildet ist, und einen zweiten Abschnitt 28b auf, der konkav ausgebildet ist. Der Abschnitt 28 weist also insgesamt eine konvex-konkave Form auf und mündet ebenfalls stetig in die Spitze 20. An der Spitze 20 schließt sich an den konkaven Bereich 28b wieder ein konvexer Bereich 21 an, der einen Teil der Spitze 20 bildet. Durch diese Form wird die Krümmung der Zentralachse Z (vgl. Fig. 7) verstärkt und gleichzeitig eine bauchige Form des Aufnahmeraums 8 gebildet. Ferner unterstützt diese Ausbildung die zipfelartige Konfiguration der Spitze 20.

Die Spitze 20 ist als verdickter Bereich der Tassenwand 4 ausgebildet. Die Tassenwand 4 weist insgesamt eine Wandstärke h₁ auf, die abgesehen von dem Rand 16 und der Spitze 20 im Wesentlichen konstant ist. Die Spitze 20 ist verdickt und weist eine maximale Dicke h₂ auf. Die maximale Dicke h₂ entspricht in etwa dem Vierfachen der Wandstärke h₁. Hierdurch ist die Spitze 20 wesentlich fester als die Tassenwand 4 in radialer Richtung.

Soll nun nach der Benutzung die Menstruationstasse 1 herausgenommen werden, kann eine Bedienerin die Menstruationstasse 1 im Bereich der Spitze 20 ergreifen.

Dabei kann die Bedienerin die Spitze 20 von beiden Seiten ergreifen. Um die Reibung zwischen den Fingern und der Menstruationstasse 1 zu erhöhen, weist die Menstruationstasse 1 im Bereich der Spitze 20 eine Strukturierung 30 auf. Die Strukturierung 30 ist ausschließlich im Bereich der Spitze angeordnet, um nur dort die Reibung zu erhöhen. Insbesondere in dem darüber liegenden Abschnitt in Richtung der Öffnung 14 der Menstruationstasse 1 ist keine Strukturierung vorgesehen; vielmehr ist die Mantelfläche 5 in diesem Bereich, in etwa den oberen zwei Dritteln oder der oberen Hälfte der Menstruationstasse, ohne Strukturierung ausgebildet. Die Mantelfläche 5 ist in diesem Bereich im Wesentlichen glatt. Hierdurch wird der Tragekomfort weiter verbessert. Es hat sich gezeigt, dass eine Strukturierung im oberen Bereich insbesondere beim Herausnehmen hinderlich sein kann, was einerseits unangenehm ist, andererseits auch leicht zum Verschütten von aufgenommener Flüssigkeit führen kann.

Die Strukturierung 30 ist als eine Mehrzahl an Vorsprüngen 32 ausgebildet, die nach Art von Kernen von Erdbeeren geformt sind. Durch diese Art der Vorsprünge 32 und der gekrümmten Form des Tassenkörpers 2 ergibt sich insgesamt die Assoziation mit einer Erdbeere der Menstruationstasse 1.

Beim Entnehmen ist es aber auch möglich, dass eine Bedienerin nicht allein die Spitze 20 greift, sondern die Finger in dem Bereich knapp hinter der Spitze 20 ansetzt, also etwas oberhalb des konkave Teilbereichs 28b, und so den Tassenkörper 2 dort zusammendrückt. Da die Tassenwand 4 flexibel ist, kann diese dort zusammengedrückt werden; der Rand 16 hält weiterhin die obere Form des Tassenkörpers 2 aufrecht, ohne zu kollabieren. Durch das Zusammendrücken der Tassenwand 4 knapp oberhalb des konkaven Teilbereichs 28b ist es möglich, die Spitze 20 zu hintergreifen, um so die Menstruationstasse 1 noch besser greifen und entnehmen zu können. Durch diesen festen Griff wird ferner nach dem Herausziehen ein Verkippen verhindert, wodurch aufgenommene Flüssigkeit in dem Aufnahmeraum 8 vorhanden bleibt und nicht verschüttet wird. Hierbei ist auch in den Figuren zu erkennen, dass die Menstruationstasse 1 gemäß der vorliegenden Erfindung keinen Stiel oder Stift im Bereich der Spitze 20 aufweist; vielmehr endet die Spitze 20 im Wesentlichen kegelförmig oder glockenförmig als konkave Noppe. Es hat sich gezeigt, dass die weit verbreiteten Stiele im Stand der Technik tendenziell dazu führen, dass die Menstruationstasse nach dem Herausnehmen verkippen kann, da die Stiele in der Regel als im Wesentlichen zylindrische Stifte ausgebildet sind und teilweise radial umlaufende Rippen aufweisen. Solche Rippen vermögen es nicht, eine Reibung zwischen Finger und Menstruationstasse derart zu erhöhen, dass eine Verkippung verhindert wird. Die einzelnen Vorsprünge 32 allerdings führen zu einer gleichmäßigen Reibungserhöhung, sowohl in axialer als auch in radialer Richtung, und dienen somit als sicherer Verkippungsschutz.

Wie sich aus den Fig. 3 und 4 ergibt, sind im oberen Bereich, knapp unterhalb des Rands 16, Belüftungslöcher 34 vorgesehen. Diese sind so klein, dass Flüssigkeit, die über den Rand 16 in den Aufnahmeraum 8 läuft, nicht durch diese hindurchtritt. Dies verhindert die Kapillarwirkung. Allerdings dienen diese Belüftungslöcher 34 dazu, einen Unterdruck auszugleichen. Wird die Menstruationstasse 1 zusammengefaltet und eingeführt, erhöht sie beim Aufspreizen durch Zurückspringen des Rings 16 das Volumen. Liegt der Rand 16 dann an der Haut an, bildet sich ein Unterdruck. Ein Unterdruck im Inneren des Aufnahmeraums 8 führt allerdings zu einer erheblich erschwerten Entnahme der Menstruationstasse und kann sich auch im Tragekomfort negativ auswirken. Die Belüftungslöcher 34 stellen eine Verbindung zwischen dem Innenraum 8 und dem Außenbereich her und können einen Druckausgleich bewirken. Hierdurch wird die Entnahme der Menstruationstasse 1 weiter vereinfacht.

Wie sich insbesondere aus Fig. 4 ergibt, ist die Menstruationstasse 1 insgesamt einstückig ausgebildet. Sie ist aus einem medizinischen Silikonmaterial mit einer Shore-Härte im Bereich von 35 bis 45 ausgebildet, insbesondere mittels Spritzgießen. Medizinisches Silikon ist besonders hautverträglich und eignet sich für diesen Einsatz. Die Shore-Härte von 35 bis 45 stellt eine ausreichende Flexibilität für den Tragekomfort dar, aber gleichzeitig eine ausreichende Steifigkeit, sodass der Rand 16 eine gewisse Formstabilität bietet und die Menstruationstasse 1 an der Spitze 20 sicher entnommen werden kann.

Alternativ zu dem medizinischen Silikon können auch herkömmliche Silikone eingesetzt werden. Bevorzugte Materialien sind insbesondere Spritzgusssilikon oder Flüssigsilikon. Insbesondere kommen Materialien wie LSR (Liquid Silicon Rubber), HTV (hochtemperaturvernetzter Silikonkautschuk), oder RTV (raumtemperaturvernetzter Silikon-Kautschuk) zum Einsatz. Auch eignen sich TPE, insbesondere weicher TPE, oder Gummi.

Die Tassenwand 4 hat in diesem Ausführungsbeispiel eine Wandstärke h1 (vgl. Fig. 4) in einem Bereich von 1,5 mm bis 2,5 mm. Der Rand 16 hat eine Wandstärke h3 von 4 bis 8 mm, in diesem Ausführungsbeispiel ca. 4,5 mm.

Vorzugsweise weist die Tassenwand 4 eine Rückstellkraft F1 (vgl. Fig. 6) gegen ein Zusammendrücken in einem Bereich von 4 N bis 15 N, vorzugsweise 5 N bis 10 N, weiter bevorzugt 6 N bis 10 N auf. Die Rückstellkraft F1 wird senkrecht zur Symmetrieebene E gemessen und zwar etwa auf halber Länge zwischen dem Rand 16 und der Spitze 20, also in etwa dem dicksten Bereich des Tassenkörpers 2. Die Öffnung 14 und etwaige Belüftungslöcher 34 bleiben dabei frei. Mit Rückstellkraft gegen ein Zusammendrücken ist die Kraft gemeint, die erforderlich ist um den Tassenkörper 2 an dieser Stelle so zusammenzudrücken, dass sich die gegenüberliegenden Seiten der Tassenwand 4 gerade eben berühren.

Ferner hat der Rand 16 der Tassenwand 4 darüber hinaus eine Rückstellkraft F2, F3 gegen ein Zusammendrücken in einem Bereich von 6 N bis 15 N. Zunächst kann eine erste Rand-Rückstellkraft F2 in der Symmetrieebene E gemessen werden, die vorzugsweise in einem Bereich von 8 N bis 15 N, vorzugsweise 9 N bis 12 N, liegen kann. Eine zweite Rand-Rückstellkraft F3 wird vorzugsweise senkrecht zur ersten Rand-Rückstellkraft F2 gemessen und liegt vorzugsweise in einem Bereich von 6 N bis 10 N, vorzugsweise 6 N bis 8 N.

Vorzugsweise sind die Rückstellkräfte F1, F2, F3 aufeinander abgestimmt, sodass beispielsweise in einem ersten Ausführungsbeispiel gilt: F1 etwa 6 N; F2 etwa 6,3 N; F3 etwa 9,6 N. In einem zweiten Ausführungsbeispiel kann gelten: F1 etwa 7,6 N; F2 etwa 7,0 N; F3 etwa 11 N. Ein drittes Ausführungsbeispiel kann die Werte aufweisen: F1 etwa 9,8 N; F2 etwa 7,3 N; F3 etwa 11,3 N. Es kann vorgesehen sein, dass bei insgesamt kleineren Tassenkörpern 2 höhere Rückstellkräfte F1, F2, F3 wirken. Zudem kann vorgesehen sein, dass die verschiedenen Ausführungsbeispiele farblich kodiert sind, sodass die Benutzerin anhand der Farbe des Tassenkörpers 2 den Bereich der Rückstellkräfte F1, F2, F3 erkennen kann.

## Patentansprüche

1. Hygieneprodukt, insbesondere Menstruationstasse (1), mit
einem Tassenkörper (2), der eine Tassenwand (4) aufweist, die mit einer inneren Oberfläche (6) einen Aufnahmeraum (8) definiert,
wobei der Tassenkörper (2) an einem ersten Ende (10) eine in den Aufnahmeraum (8) mündende Öffnung (14) mit einem Rand (16) und an einem zweiten Ende (12) einen der Öffnung (14) gegenüberliegenden Boden (18) aufweist, und
wobei der Tassenkörper (2) eine im Allgemeinen kegelige Form aufweist, sich zum Boden (18) hin verjüngt und in einer Spitze (20) endet,
**dadurch gekennzeichnet, dass** der Tassenkörper (2) rotationsunsymmetrisch ist und eine Zentralachse (Z) des Tassenkörpers (2) gekrümmt verläuft.

2. Hygieneprodukt nach Anspruch 1, wobei der Tassenkörper (2) eine Symmetrieebene (E) aufweist und bezogen auf diese spiegelsymmetrisch ausgebildet ist.

3. Hygieneprodukt nach Anspruch 2, wobei ein erster Abschnitt (26) der Tassenwand (4) entlang der Symmetrieebene (E) von dem Rand (16) der Öffnung (14) bis zur Spitze (20) an der Außenseite im Wesentlichen teilkreisförmig verläuft.

4. Hygieneprodukt nach Anspruch 2 oder 3, wobei ein zweiter Abschnitt (28) der Tassenwand (4) entlang der Symmetrieebene (E) von dem Rand (16) der Öffnung (14) bis zur Spitze (20) im Wesentlichen eine konvex-konkave Form definiert.

5. Hygieneprodukt nach einem der vorstehenden Ansprüche, wobei der Tassenkörper (4) eine bauchige Form hat, indem sich der Tassenkörper (4) im Bereich der Öffnung (14) leicht verjüngt.

6. Hygieneprodukt nach einem der vorstehenden Ansprüche, wobei die Spitze (20) eine im Allgemeinen kegelige Form hat, die als Verdickung in der Tassenwand (4) ausgebildet ist.

7. Hygieneprodukt nach Anspruch 6, wobei die Tassenwand (4) knickfrei in die Spitze (20) übergeht.

8. Hygieneprodukt nach einem der vorstehenden Ansprüche, wobei die Tassenwand (4) äußerlich im Bereich der Spitze (20) eine Strukturierung (30) aufweist.

9. Hygieneprodukt nach einem der vorstehenden Ansprüche, wobei der Rand (16) der Öffnung (14) eine Verstärkung aufweist, die vorzugsweise als Verdickung der Tassenwand (4) ausgebildet ist.

10. Hygieneprodukt nach einem der vorstehenden Ansprüche, wobei der Rand (16) der Öffnung (14) zum Aufnahmeraum (8) hin schräg abfällt.

11. Hygieneprodukt nach einem der vorstehenden Ansprüche, wobei die Tassenwand (4) zwischen dem Rand (16) der Öffnung und der Spitze (20) einem im Wesentlichen konstante Wandstärke (h₁) aufweist.

12. Hygieneprodukt nach einem der vorstehenden Ansprüche, wobei der Tassenkörper (2) aus einem Silikon, vorzugsweise medizinischem Silikon, Spitzgusssilikon oder Flüssigsilikon, mit einer Shore-Härte in einem Bereich von 18 bis 60, insbesondere 30 bis 50, vorzugsweise 35 bis 45 gebildet ist.

13. Hygieneprodukt nach einem der vorstehenden Ansprüche, wobei die Tassenwand (4) eine Wandstärke (h1) in einem Bereich von 0,8 mm bis 8,0 mm, vorzugsweise 1,0 mm bis 3,0 mm, weiter vorzugsweise 1,5 mm bis 2,5 mm aufweist.

14. Hygieneprodukt nach einem der vorstehenden Ansprüche, wobei die Tassenwand (4) eine Rückstellkraft gegen ein Zusammendrücken in einem Bereich von 4 N bis 15 N, vorzugsweise 5 N bis 10 N, weiter bevorzugt 6 N bis 10 N aufweist.

15. Hygieneprodukt nach einem der vorstehenden Ansprüche, wobei der Rand (16) eine Rückstellkraft gegen ein Zusammendrücken in einem Bereich von 6 N bis 15 N aufweist.

## Claims

1. Hygiene product, in particular a menstrual cup (1), with a cup body (2) which has a cup wall (4) which, with an inner surface (6), defines a receiving space (8), the cup body (2) having, at a first end (10), an opening (14) which opens into the receiving space (8) and has an edge (16) and, at a second end (12), a bottom (18) opposite the opening (14), and the cup body (2) having a generally conical shape, tapering towards the bottom (18), and ending in a tip (20), **characterised in that** the cup body (2) is rotationally asymmetrical and a central axis (Z) of the cup body (2) is curved.

2. Hygiene product according to claim 1, wherein the cup body (2) has a plane of symmetry (E) and is mirror-symmetrical with respect thereto.

3. Hygiene product according to claim 2, wherein a first section (26) of the cup wall (4) extends in a substantially partially circular manner along the plane of symmetry (E) from the edge (16) of the opening (14) to the tip (20) on the outside.

4. Hygiene product according to claim 2 or 3, wherein a second section (28) of the cup wall (4) defines a substantially convex-concave shape along the plane of symmetry (E) from the edge (16) of the opening (14) to the tip (20).

5. Hygiene product according to one of the preceding claims, wherein the cup body (4) has a bulbous shape in that the cup body (4) tapers slightly in the region of the opening (14).

6. Hygiene product according to one of the preceding claims, wherein the tip (20) has a generally conical shape which is formed as a thickening in the cup wall (4).

7. Hygiene product according to claim 6, wherein the cup wall (4) merges into the tip (20) without kinks.

8. Hygiene product according to one of the preceding claims, wherein the cup wall (4) has a structure (30) on the outside in the region of the tip (20).

9. Hygiene product according to one of the preceding claims, wherein the edge (16) of the opening (14) has a reinforcement, which is preferably formed as a thickening of the cup wall (4).

10. Hygiene product according to one of the preceding claims, wherein the edge (16) of the opening (14) slopes down towards the receiving space (8).

11. Hygiene product according to one of the preceding claims, wherein the cup wall (4) has a substantially constant wall thickness (h₁) between the edge (16) of the opening and the tip (20).

12. Hygiene product according to one of the preceding claims, wherein the cup body (2) is made from a silicone, preferably medical silicone, injection-moulded silicone or liquid silicone, with a Shore hardness in a range from 18 to 60, in particular 30 to 50, preferably 35 to 45.

13. Hygiene product according to one of the preceding claims, wherein the cup wall (4) has a wall thickness (h1) in a range from 0.8 mm to 8.0 mm, preferably 1.0 mm to 3.0 mm, further preferably 1.5 mm to 2.5 mm.

14. Hygiene product according to one of the preceding claims, wherein the cup wall (4) has a restoring force against compression in a range from 4 N to 15 N, preferably 5 N to 10 N, more preferably 6 N to 10 N.

15. Hygiene product according to one of the preceding claims, wherein the edge (16) has a restoring force against compression in a range from 6 N to 15 N.

## Revendications

1. Produit hygiénique, notamment coupe (1) menstruelle, comprenant
un corps (2) de coupe, qui a une paroi (4) de coupe, définissant, avec une surface (6) intérieure, un espace (8) de réception,
dans lequel le corps (2) de la coupe a, à une première extrémité (10), une ouverture (14) débouchant dans l'espace (8) de réception et ayant un bord (16) et, à une deuxième extrémité (12), un fond (18) faisant face à l'ouverture (14) et
dans lequel le corps (2) de la coupe a une forme conique d'une manière générale, qui se rétrécit vers le fond (18) et se termine en une pointe (20),
**caractérisé en ce que** le corps (2) de la coupe n'est pas de révolution et un axe (Z) central du corps (2) de la coupe s'étend de manière incurvée.

2. Produit hygiénique suivant la revendication 1, dans lequel le corps (2) de la coupe a un plan (E) de symétrie et est constitué d'une manière symétrique comme en un miroir par rapport à celui-ci.

3. Produit hygiénique suivant la revendication 2, dans lequel une première partie (26) de la paroi (4) de la coupe s'étend sensiblement en forme de partie de cercle du côté extérieur, le long du plan (E) de symétrie, du bord (16) de l'ouverture (14) jusqu'à la pointe (20).

4. Produit hygiénique suivant la revendication 2 ou 3, dans lequel une deuxième partie (28) de la paroi (4) de la coupe définit sensiblement une forme convexe-concave, le long du plan (E) de symétrie, du bord (16) de l'ouverture (14) jusqu'à la pointe (20).

5. Produit hygiénique suivant l'une des revendications précédentes, dans lequel le corps (4) de la coupe a une forme ventrue par le fait que le corps (4) de la coupe se rétrécit légèrement dans la région de l'ouverture (14).

6. Produit hygiénique suivant l'une des revendications précédentes, dans lequel la pointe (20) a, d'une manière générale, une forme conique constituée sous la forme d'une épaisseur dans la paroi (4) de la coupe.

7. Produit hygiénique suivant la revendication 6, dans lequel la paroi (4) de la coupe se transforme sans coude en la pointe (20).

8. Produit hygiénique suivant l'une des revendications précédentes, dans lequel la paroi (4) de la coupe a, à l'extérieur, une structuration (30) dans la région de la pointe (20).

9. Produit hygiénique suivant l'une des revendications précédentes, dans lequel le bord (16) de l'ouverture (14) a un renfort constitué de préférence sous la forme d'un épaississement de la paroi (4) de la coupe.

10. Produit hygiénique suivant l'une des revendications précédentes, dans lequel le bord (16) de l'ouverture (14) descend de manière inclinée vers l'espace (8) de réception.

11. Produit hygiénique suivant l'une des revendications précédentes, dans lequel la paroi (4) de la coupe a une épaisseur (h₁) de paroi sensiblement constante entre le bord (16) de l'ouverture et la pointe (20).

12. Produit hygiénique suivant l'une des revendications précédentes, dans lequel le corps (2) de la coupe est en silicone, de préférence en silicone médical, en silicone moulé par injection ou en silicone liquide ayant une dureté Shore dans une plage de 18 à 60, notamment de 30 à 50, de préférence de 35 à 45.

13. Produit hygiénique suivant l'une des revendications précédentes, dans lequel la paroi (4) de la coupe a une épaisseur (h₁) de paroi dans une plage de 0,8 mm à 8,0 mm, de préférence de 1,0 mm à 3,0 mm, d'une manière encore plus préférée, de 1,5 mm à 2,5 mm.

14. Produit hygiénique suivant l'une des revendications précédentes, dans lequel la paroi (4) de la coupe a une force de rappel à l'encontre d'une compression dans une plage 4 N à 15 N, de préférence de 5 N à 10 N, d'une manière encore plus préférée, de 6 N à 10 N.

15. Produit hygiénique suivant l'une des revendications précédentes, dans lequel le bord (16) a une force de rappel vis-à-vis d'une compression dans une plage de 6 N à 15 N.
